# EUROPEAN PATENT SPECIFICATION

(11) **EP 1 946 110 B1**
(45) Date of publication and mention of the grant of the patent: **06.10.2010**
(21) Application number: 06823396.4
(22) Date of filing: 08.11.2006
(51) Int. Cl.: G01N 33/566, C07K 5/00, A23L 1/48

(54) **SCREENING METHOD FOR KOKUMI-IMPARTING AGENTS**
SCREENING-VERFAHREN FÜR KOKUMI-VERMITTELNDE AGENTIEN
MÉTHODE DE DISPISTAGE POUR DES AGENTS DE DISTRIBUTION KOKUMI

(30) Priority: 09.11.2005 JP 2005325300; 22.11.2005 US 738562 P; 07.07.2006 JP 2006188458; 20.07.2006 US 807831 P
(43) Date of publication of application: 23.07.2008
(60) Divisional application: 09007706.6
(73) Proprietor: Ajinomoto Co., Inc., Tokyo 104-8315 (JP)
(72) Inventor: OHSU, Takeaki, Kawasaki-shi, Kanagawa, 210-8681 (JP); TAKESHITA, Sen, Kawasaki-shi, Kanagawa, 210-8681 (JP); ETO, Yuzuru, Kawasaki-shi, Kanagawa, 210-8681 (JP); AMINO, Yusuke, Kawasaki-shi, Kanagawa, 210-8681 (JP); MIYAMURA, Naohiro, Kawasaki-shi, Kanagawa, 210-8681 (JP); YAMANAKA, Tomohiko, Kawasaki-shi, Kanagawa, 210-8681 (JP); NAGASAKI, Hiroaki, Kawasaki-shi, Kanagawa, 210-8681 (JP)
(74) Representative: Strehl Schübel-Hopf & Partner
(86) International application number: PCT/JP2006/322694
(87) International publication number: WO 2007/055393

(56) References cited:
- EP-A- 1 554 939
- EP-A1- 0 672 354
- WO-A-92/07267
- WO-A-94/22438
- UEDA Y. ET AL: AGRI BIOL CHEM, vol. 54, no. 1, 1990, pages 163-169, AM CHEM SOC ST LOUIS MI USA
- UEDA Y. ET AL: BIOSCI. BIOTECH. BIOCHEM., vol. 58, no. 1, 1994, pages 108-110, AM CHEM SOC ST LOUIS MI USA
- DUNKEL A. ET AL: J. AGRIC. FOOD CHEM., vol. 55, 2007, pages 6712-6719, AM CHEM SOC ST LOUIS MI USA
- UEDA Y. ET AL: AGRI BIOL CHEM, vol. 54, no. 1, 1990, pages 163-169, AM CHEM SOC ST LOUIS MI USA
- UEDA Y. ET AL: BIOSCI. BIOTECH. BIOCHEM., vol. 58, no. 1, 1994, pages 108-110, AM CHEM SOC ST LOUIS MI USA
- DUNKEL A. ET AL: J. AGRIC. FOOD CHEM., vol. 55, 2007, pages 6712-6719, AM CHEM SOC ST LOUIS MI USA

## Description

### Technical Field

The present invention relates to a method for screening for a kokumi-imparting substance.

### Background Art

The calcium receptor is also called the calcium sensing receptor (CaSR), and is a.receptor consisting of 1078 amino acids, and is classified into the class C of seven-transmembrane receptors (G protein-coupled receptor; GPCR). Cloning of the gene for this calcium receptor was reported in 1993 (Non-patent document 1), and it is known to cause various cell responses through elevation of intracellular calcium level etc., when it is activated with calcium etc. The gene sequence of the human calcium receptor is registered with GenBank Accession No. NM_000388, and is well conserved in animals.

The aforementioned calcium receptor may act to promote or suppress biological functions. Therefore, at present, therapeutic agents which may act as activators and inhibitors of the calcium receptor are appropriately used in the treatment of neurological diseases, hepatic diseases, cardiovascular diseases, digestive system diseases, and other diseases, depending on pathological conditions. For example, the calcium receptor is able to detect increased blood calcium level in the parathyroid, and suppress secretion of the parathyroid hormone (PTH) to correct the blood calcium level. Therefore, an effect of reducing the blood calcium level is expected for a calcium receptor activator. It has been actually clarified that when a calcium receptor activator is used to treat secondary hyperparathyroidism in a hemodialysis patient, it reduces the PTH level without elevating the calcium and phosphorus levels.

Since functional analysis of the calcium receptor has been conducted mainly for calcium homeostasis, the applied researches therefore are so far mainly concerned bone metabolic diseases in which calcium regulation is involved. However, it has become clear that the calcium receptor is widely distributed in living bodies other than the parathyroid and kidney from the results of genetic expression analyses etc. (Non-patent documents 2 and 3), and possibilities thereof for being involved in various biological functions and causes of diseases have been proposed. For example, it is estimated that the calcium receptor is involved in the functions of the liver, heart, lung, alimentary canal, lymphocyte, and pancreas. The inventors of the present invention also have confirmed that it is expressed in a wide range of tissues in living bodies by analyses based on RT-PCR using RNAs extracted from rat tissues. From the aforementioned viewpoints, the values of activators and inhibitors of the calcium receptor are presently rapidly increasing for applications.

Moreover, other than calcium, cations such as gadolinium cation, basic peptides such as polyarginine, polyamine such as spermine, amino acids such as phenylalanine, and so forth have been reported as calcium receptor activators (Non-patent document 4).

Although many specific activators have been developed so far as calcium receptor activators as described above, there are few compounds existing in living bodies among them, and the activities of the compounds existing in living bodies are very low. Therefore, therapeutic agents for various diseases containing these activators have serious problems concerning side reaction, permeability and activity. For example, although it is known that amino acids act on the calcium receptors, it is considered that actual application thereof as the activators is difficult since the activity is very weak. Moreover, although macromolecules such as polyarginine have been reported as the activator as described above, it is estimated that the functions are based on the actions as polyvalent cations having irregular structures. That is, it is not known that a peptide having a specific structure is useful as a calcium receptor activator.

Meanwhile, in the field of foodstuffs, taste substances have been applied for many years. In particular, substances having the five basic tastes, namely, sweet taste, salty taste, sour taste, bitter taste, and umami (delicious taste), and substances enhancing these have widely been used as seasonings. As a concept of taste that cannot be expressed with the aforementioned tastes, there is "kokumi". Kokumi means a taste that cannot be expressed with the five basic tastes, and means a taste that enhances not only the basic tastes but also marginal tastes of the basic tastes, such as thickness, growth (mouthfulness), continuity, and harmony. Several methods for imparting kokumi have been reported so far, and glutathione (Patent document 1), heated products of gelatin and tropomyosin (Patent document 2), sulfone group-containing compounds (Patent document 3), a peptide containing the Asn-His sequence (Patent document 4), and so forth have been reported.

Although development of various kokumi-imparting substances has been attempted as described above, and commercialization has been made mainly for extracts of natural products, there are presently very few examples of isolation of a pure kokumi component from an extract of natural product, such as glutathione and N-(4-methyl-5-oxo-1-imidazolin-2-yl)sarcosine.

Therefore, the development of highly effective, safe and inexpensive kokumi-imparting substances is desired, and a convenient and highly sensitive method for screening for a kokumi-imparting substance is needed for that purpose.
[Non-patent document 1] Nature, 1993 Dec 9;366(6455):575-80
[Non-patent document 2] J. Endocrinol., 2000 May, 165 (2):173-7
[Non-patent document 3] Eur. J. Pharmacol., 2002 Jul. 5, 447(2-3):271-8
[Non-patent document 4] Cell Calcium., 2004 Mar., 35 (3) :209-16
[Patent document 1] Japanese Patent No. 1464928
[Patent document 2] Japanese Patent Laid-open Publication (KOKAI) No. 10-276709
[Patent document 3] Japanese Patent Laid-open Publication (KOKAI) No. 8-289760
[Patent document 4] WO2004/096836

EP-A-1 554 939 discloses the provision of kokumi-imparting substances such as amino acids and peptides.

EP-A1-0 672 354 discloses a kokumi-imparting mixture containing peptides and low-molecular weight fractions of natural extracts.

WO 94/22438 A discloses compositions containing glutathione and a calcium salt.

WO 92/07267 A discloses a composition comprising peptides.

### Disclosure of Invention

### [Problems to be solved by the Invention]

An object of the present invention is to provide a convenient and highly sensitive method for screening for a kokumi-imparting substance.

### [Means for solving the Problems]

The present invention provides the following.
(1) A method for screening for a kokumi-imparting substance, i.e a substance capable of enhancing salty taste, umami, sweet taste, sour taste or bitter taste, which comprises the first step of reacting a calcium receptor and a test substance and detecting calcium receptor activity, and the second step of measuring the kokumi-imparting effect of the test substances for which calcium receptor activation activity is.detected in the first step.

### Effect of the Invention

According to the present invention, a convenient and highly sensitive method for screening for a kokumi-imparting substance is provided.

### Brief Description of the Drawings

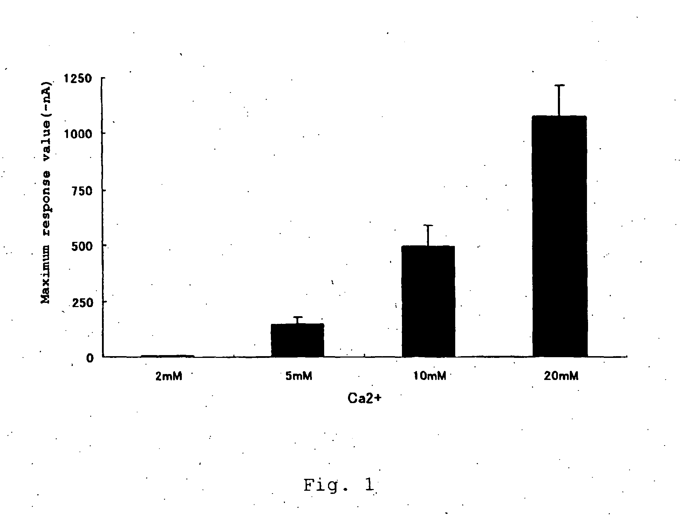

[Fig. 1] Drawing showing an action of calcium on the calcium receptor.
   The human calcium receptor cRNA was introduced into *Xenopus laevis* oocytes by microinjection. Recorded were values of intracellular response currents which flew when a calcium chloride solution was added at an arbitrary concentration. The maximum values of intracellular currents were considered response current values. It was confirmed that no response was observed in oocytes introduced with distilled water.by microinjection as a control.

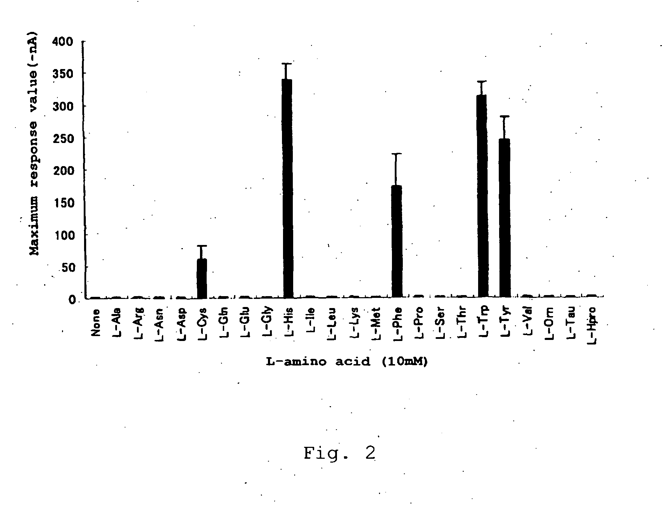

[Fig. 2] Drawing showing an action of L-amino acids on the calcium receptor.
   The human calcium receptor cRNA was introduced into *Xenopus laevis* oocytes by microinjection. Recorded were values of intracellular response currents which flew when a 10 mM L-amino acid solution was added. The maximum values of intracellular currents were considered response current values. It was confirmed that no response was observed in oocytes introduced with distilled water by microinjection as a control.

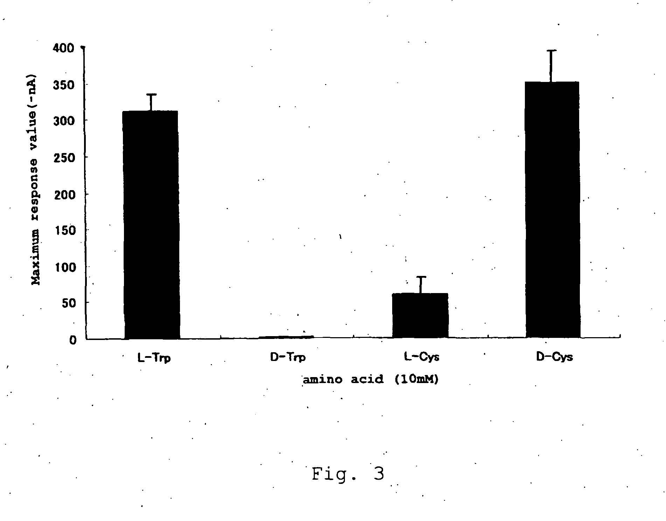

[Fig. 3] Drawing showing an action of D-amino acids on the calcium receptor.
   The human calcium receptor cRNA was introduced into *Xenopus laevis* oocytes by microinjection. Recorded were values of intracellular response currents which flew when a 10 mM D-amino acid solution was added. The maximum values of intracellular currents were considered response current values. It was confirmed that no response was observed in oocytes introduced with distilled water by microinjection as a control.

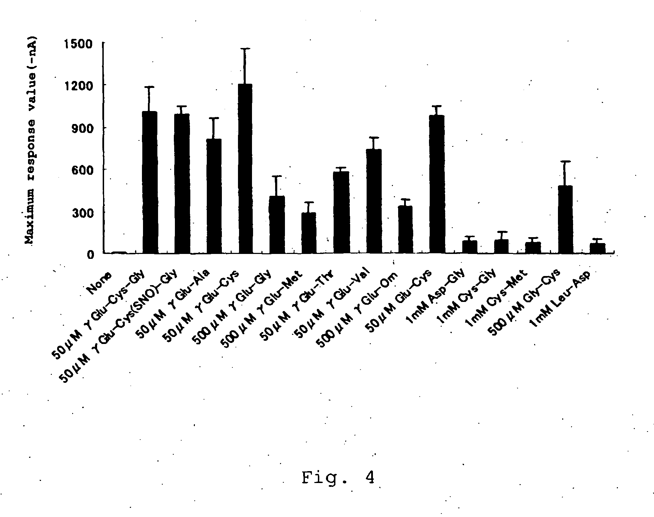

[Fig. 4] Drawing showing an action of peptides on the calcium receptor.
   The human calcium receptor cRNA was introduced into *Xenopus laevis* oocytes by microinjection. Recorded were values of intracellular response currents which flew when a peptide solution was added at an arbitrary concentration. The maximum values of intracellular currents were considered response current values. It was confirmed that no response was observed in oocytes introduced with distilled water by microinjection as a control.

### Best Mode for Carrying Out the Invention

Hereafter, the present invention will be explained in detail.

In this specification, the "calcium receptor" is also called the calcium sensing receptor (CaSR), and belongs to the class C of seven-transmembrane receptors. In this specification, the "calcium receptor activity" means binding to the aforementioned calcium receptor to activate the guanine nucleotide binding protein and thereby transmit signals. Furthermore, in this specification, the "calcium receptor activator" is a substance that acts on the aforementioned calcium receptor to activate the calcium receptor and control the functions of cells expressing the calcium receptor.

In this specification, "kokumi" means a taste that cannot be expressed by the five basic tastes, sweet taste, salty taste, sour taste, bitter taste, and umami, in which, not only the basic tastes, marginal tastes of the basic tastes, such as thickness, growth (mouthfulness), continuity, and harmony are enhanced. Further, the "kokumi-imparting agent" or "kokumi-imparting substance" refers to an agent or substance that can enhance the five basic tastes, sweet taste, salty taste, sour taste, bitter taste, and umami, and impart marginal tastes of the basic tastes, such as thickness, growth (mouthfulness), continuity, and harmony accompanying the basic tastes. Therefore, the kokumi-imparting agent can also be used as a sweet taste-enhancing agent, salty taste-enhancing agent, sour taste-enhancing agent, bitter taste-enhancing agent, or umami-enhancing agent. As for intensity of kokumi, "first and middle taste" means taste that is felt during the period of 0 to 4 seconds after eating, and "aftertaste" means taste that is felt after 5 seconds following eating.

In this specification, all the amino acids and amino acid residues constituting the peptides are L-isomers, unless otherwise specified.

### <1> Method for screening for kokumi-imparting substance

The method for screening for a kokumi-imparting substance of the present invention (henceforth also referred to as the screening method of the present invention) is characterized by using the calcium receptor activity as an index. Specifically, the screening method of the present invention comprises the first step of reacting a calcium receptor and a test substance and detecting calcium receptor activity, and the second step of measuring the kokumi-imparting effect of the test substances for which calcium receptor activation activity is detected in the first step.

The specific process steps of the screening method of the present invention are exemplified below. However, the steps of the screening method of the present invention are not limited to these steps.
1) A test substance is added to a calcium receptor activity measurement system for measuring the calcium receptor activity, and the calcium receptor activity is measured.
2) The calcium receptor activity obtained with adding the test substance and the calcium receptor activity obtained without adding the test substance are compared.
3) A test substance showing a higher calcium receptor-stimulating activity when the test substance is added is chosen.
4) The kokumi-imparting effect of the chosen test substance is measured, and a test substance having a kokumi-imparting effect is chosen.

The calcium receptor activity is measured by using, for example, a measurement system using cells expressing the calcium receptor. The cells may be cells endogenously expressing the calcium receptor, or recombinant cells introduced with a foreign calcium receptor gene. As the aforementioned calcium receptor activity measurement system, any system may be used without particular limitation so long as chosen is a system with which when an extracellular ligand (activator) specific to the calcium receptor is added to the cells expressing the calcium receptor, binding (reaction) of the activator and the calcium receptor can be detected, or a detectable signal is transmitted into cells in response to binding (reaction) of the activator and the calcium receptor. When the reaction of tested compound result calcium receptor activity; such tested compound are determined as having calcium receptor activation activity and kokumi-imparting compound.

The aforementioned kokumi-imparting effect can be confirmed by a taste test by a human, or the like. Furthermore, the test substance used for the screening is not particularly limited, and low molecular compounds, saccharides, peptides, proteins, and so forth can be used.

As the aforementioned calcium receptor, the human calcium receptor encoded by the human calcium receptor gene registered with GenBank Accession No. NM_000388 can be exemplified as a preferred example. In addition, the calcium receptor is not limited to the protein encoded by the gene of the aforementioned sequence, and it may be a protein encoded by a gene having a homology of 60% or more, preferably 80% or more, more preferably 90% or more, to the aforementioned sequence, so long as the protein has the calcium receptor function. The GPRC6A receptor and the 5.24 receptor are also known as subtypes of the calcium receptor, and they can be used for the present invention. The calcium receptor function can be examined by expressing a gene of interest in a cell and measuring changes in the electric current, or intracellular calcium ion concentration at the time of the addition of calcium.

The origin of the calcium receptor is not particularly limited, and examples include, besides the aforementioned human calcium receptor, calcium receptors derived from animals such as mouse, rat, and dog.

As described above, the calcium receptor activity can be confirmed by using live cells which express a calcium receptor or a fragment thereof, cell membranes which express a calcium receptor or a fragment thereof, an in vitro system containing a protein of a calcium receptor or a fragment thereof, or the like.

An example using live cells is shown below. However, confirmation of the calcium receptor activity is not limited to this example.

The calcium receptor can be expressed in cultured cells such as *Xenopus laevis* oocytes, hamster ovarian cells, and human fetal kidney cells. The calcium receptor can be expressed by cloning a calcium receptor gene in a plasmid that can contain a foreign gene and introducing the plasmid or cRNA obtained by using the plasmid as a template. To detect the reaction, electrophysiological techniques, fluorescent indicator reagents that indicate elevation of intracellular calcium level, and so forth, can be used.

Expression of the calcium receptor is first confirmed based on the response to calcium or a specific activator. Oocytes that showed intracellular current with calcium at a concentration of about 5 mM or cultured cells that showed fluorescence of the fluorescent indicator reagent with calcium at a concentration of about 5 mM are used. Calcium concentration dependency is determined by changing the calcium concentration. Then, a test substance such as a peptide is prepared to a concentration of about 1 µM to 1 mM, and added to the oocytes or cultured cells, and the calcium receptor activity of the test substance such as the aforementioned peptide is measured.

### Examples

Hereafter, the present invention will be more specifically explained with reference to examples.

### <Example 1> (reference only)

### <Preparation of gene (cRNA)>

The gene of the calcium receptor was prepared as follows. On the basis of the DNA sequence registered at NCBI (calcium receptor: NM_000388), synthetic oligo DNAs (forward primer (N) and reverse primer (C)) used for PCR were designed (Table 1, SEQ ID NOS: 1 and 2).

**Table 1**

| Synthetic oligo DNAs (forward primer (N) and reverse primer (C), h: human) | |
|---|---|
| Code | Sequence(5'-3') |
| hCASR_N | ACTAATACGACTCACTATAGGGACCATGGCATTTTATAGCTGCTGCTGG |
| hCASR_C | TTATGAATTCACTACGTTTTCTGTAACAG |

By using human kidney cDNA (Clontech) as a material, the primers shown in Table 1 (hCASR_N (SEQ ID NO: 1) and hCASR_C (SEQ ID NO: 2)) were synthesized, and PCR was performed by using Pfu ultra DNA Polymerase (Stratagene) under the following conditions. After a reaction at 94°C for 3 minutes, a cycle of reactions at 94°C for 30 seconds, 55°C for 30 seconds, and 72°C for 2 minutes was repeated 35 times, and then a reaction was performed at 72°C for 7 minutes. Wether amplification was attained by PCR was detected by performing agarose electrophoresis, staining with a DNA staining reagent, and ultraviolet irradiation. The chain lengths of the PCR products were confirmed by comparison with DNA markers of known sizes simultaneously subjected to the electrophoresis. The plasmid vector pBR322 (Takara) was digested with the restriction enzyme EcoRV. The gene fragment amplified by PCR was ligated to the cleavage site of the plasmid by using Ligation Kit (Promega). The *Escherichia coli* DH5α strain was transformed with each ligation reaction solution, and a transformant harboring the plasmid in which the PCR amplification product was cloned was selected. The PCR amplification product was confirmed by DNA sequence analysis. By using this recombinant plasmid as a template together with a cRNA preparation kit (Ambion), cRNA of the calcium receptor gene was prepared.

### <Example 2> (reference only)

### <Preparation of various samples>

As L-amino acid samples, 23 kinds of special grade amino acids, alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, ornithine, taurine (all of these from Ajinomoto), and hydroxyproline (Nakarai Tesque), were used. As D-Cys and D-Trp (Nakarai Tesque) and calcium chloride, those of special grade were used. Furthermore, as peptide samples, γ-Glu-Cys-Gly (Sigma Aldrich Japan), γ-Glu-Cys(SNO)-Gly (Dojin Chemical Laboratory), γ-Glu-Ala (Bachem Feinchemikalien AG), γ-Glu-Gly (Bachem Feinchemikalien AG), γ-Glu-Cys (Sigma Aldrich Japan), γ-Glu-Met (Bachem Feinchemikalien AG), γ-Glu-Abu-Gly (Abu: α-aminobutyric acid, Bachem Feinchemikalien AG), γ-Glu-Thr (Kokusan Chemical), γ-Glu-Val (Kokusan Chemical), γ-Glu-Leu (contract manufactured product), γ-Glu-Ile (contract manufactured product), γ-Glu-Orn (Kokusan Chemical), Asp-Gly (contract manufactured product), Cys-Gly (contract manufactured product), Cys-Met (contract manufactured product), Glu-Cys (contract manufactured product), Gly-Cys (contract manufactured product), Leu-Asp (contract manufactured product), γ-Glu-Val-Val (contract manufactured product), γ-Glu-Val-Glu (contract manufactured product), γ-Glu-Val-Lys (contract manufactured product), γ-Glu-y-Glu-Val (contract manufactured product), γ-Glu-Gly-Gly (contract manufactured product), γ-Glu-Val-Phe (contract manufactured product), γ-Glu-Val-Ser (contract manufactured product), γ-Glu-Val-Pro (contract manufactured product) γ-Glu-Val-Arg(contract manufactured product), γ-Glu-Val-Asp(contract manufactured product), γ-Glu-Val-Met(contract manufactured product), γ-Glu-Val-Thr(contract manufactured product), γ-Glu-Val-His(contract manufactured product), γ-Glu-Val-Asn(contract manufactured product.), γ-Glu-Val-Gln(contract manufactured product), γ-Glu-Val-Cys(contract manufactured product), γ-Glu-Val-Orn(contract manufactured product) andγ-Glu-Ser-Gly(contract manufactured product) were used. Glutamine and cysteine were prepared upon use, and the other samples were stored at -20°C after preparation. As the peptides, those having a purity of 90% or higher were used.' As only for γ-Glu-Cys, one having a purity of 80% or higher was used. When the solution dissolving each sample showed an acidic or alkaline pH, the solution was adjusted to an approximately neutral pH by using NaOH or HCl. The solution used for dissolution of amino acids and peptides, preparation of *Xenopus laevis* oocytes, and culture of the oocytes had the following composition: 96 mM NaCl, 2 mM KCl, 1 mM MgCl₂, 1.8 mM CaCl₂, 5 mM Hepes, pH 7.2.

### <Example 3> (reference only)

### <Synthesis of γ-Glu-Val-Gly>

Boc-Val-OH (8.69 g, 40.0 mmol) and Gly-OBzl·HCl (8.07 g, 40.0 mmol) were dissolved in methylene chloride (100 ml), and the solution was kept at 0°C. Triethylamine (6.13 ml, 44.0 mmol), HOBt (1-hydroxybenzotriazole, 6.74 g, 44.0 mmol), and WSC·HCl (1-ethyl-3-(3-dimethylaminopropyl) carbodiimide hydrochloride; 8.44 g, 44.0 mmol) were added to the solution, and the mixture was stirred overnight at room temperature. The reaction mixture was concentrated under reduced pressure, and the residue was dissolved in ethyl acetate (200 ml). The solution was washed with water (50 ml), 5% citric acid aqueous solution (50 ml x twice), saturated brine (50 ml), 5% sodium hydrogencarbonate aqueous solution (50 ml x twice), and saturated brine (50 ml). The organic layer was dried over anhydrous magnesium sulfate, magnesium sulfate was removed by filtration, and the filtrate was concentrated under reduced pressure. The residue was recrystallized from ethyl acetate/n-hexane to obtain Boc-Val-Gly-OBzl (13.2 g, 36.2 mmol) as white crystals.

Boc-Val-Gly-OBzl (5.47 g, 15.0 mmol) was added to 4 N HCl/dioxane solution (40 ml), and the mixture was stirred at room temperature for 50 minutes. Dioxane was removed by concentration under reduced pressure, n-hexane (30 ml) was added to the residue, and the mixture was concentrated under reduced pressure. This procedure was repeated 3 times to quantitatively obtain H-Val-Gly-OBzl·HCl.

The above H-Val-Gly-OBzl·HCl and Z-Glu-OBzl (5.57 g, 15.0 mmol) were dissolved in methylene chloride (50 ml), and the solution was kept at 0°C. Triethylamine (2.30 ml, 16.5 mmol), HOBt (1-hydroxybenzotriazole, 2.53 g, 16.5 mmol), and WSC·HCl (1-ethyl-3-(3-dimethylaminopropyl) carbodiimide hydrochloride, 3.16 g, 16.5 mmol) were added to the solution, and the mixture was stirred at room temperature for 2 days. The reaction mixture was concentrated under reduced pressure, and the residue was dissolved in heated ethyl acetate (1500 ml). The solution was washed with water (200 ml), 5% citric acid aqueous solution (200 ml x twice), saturated brine (150 ml), 5% sodium hydrogencarbonate aqueous solution (200 ml x twice), and saturated brine (150 ml). The organic layer was dried over anhydrous magnesium sulfate, magnesium sulfate was removed by filtration, and the filtrate was concentrated under reduced pressure. The deposited crystals were collected by filtration, and dried under reduced pressure to obtain Z-Glu(Val-Gly-OBzl)-OBzl (6.51 g, 10.5 mmol) as white crystals.

The above Z-Glu(Val-Gly-OBzl)-OBzl (6.20 g, 10.03 mmol) was suspended in ethanol (200 ml), and added with 10% palladium/carbon (1.50 g), and reduction reaction was performed at 55°C for 5 hours under a hydrogen atmosphere. During the reaction, 100 ml in total of water was gradually added. The catalyst was removed by filtration using a Kiriyama funnel, and the filtrate was concentrated under reduced pressure to a half volume. The reaction mixture was further filtered through a membrane filter, and the filtrate was concentrated under reduced pressure.' The residue was dissolved in a small volume of water, and added with ethanol to deposit crystals, and the crystals were collected by filtration, and dried under reduced pressure to obtain white powder of γ-Glu-Val-Gly (2.85 g, 9.40 mmol).. ESI-MS:(M+H)⁺ = 304.1
¹H-NMR (400 MHz, D₂O) δ (ppm): 0.87 (3H, d, J=6.8 Hz), 0.88 (3H, d, J=6.8 Hz), 1.99-2.09 (3H, m), 2.38-2.51 (2H, m) 3.72 (1H, t, J=6.35 Hz), 3.86 (1H, d, J=17.8 Hz), 3.80 (1H, d, J=17.8 Hz), 4.07 (1H, d, J=6.8 Hz)

### <Example 4> (reference only)

### <Synthesis of γ-Glu-Cys(S-Me)-Gly [Cys(S-Me): S-methylcysteine]>

Reduced glutathione (15.0 g, 48.8 mmol) was added to water (45 ml), and sodium hydroxide (4.52 g, 2.2 equivalents, 107 mmol) was added portionwise to the mixture while the mixture was bubbled with nitrogen. Methyl iodide (4.56 ml, 1.5 equivalents, 73 mmol) was added to the mixture, and the mixture was sealed and stirred at room temperature for 2 hours. The reaction mixture.was adjusted to pH 2 to 3 with concentrated hydrochloric acid, added with ethanol (150 ml), and stored overnight in a refrigerator. Since oily matter was separated, the supernatant was removed. When the remained oily matter was dissolved in water and gradually added with ethanol, partially crystallized oily matter was deposited.

Therefore, the supernatant liquid was removed again. The residue was dissolved in water (300 ml), adsorbed on an ion exchange resin (Dowex 1-acetate, 400 ml) filled in a column, and after washing with water, eluted with 1 N acetic acid aqueous solution. The eluate was concentrated under reduced pressure, and precipitated from water/ethanol to obtain a white powder of γ-Glu-Cys(S-Me)-Gly (5.08 g, 15.8 mmol).
FAB-MS: (M+H)⁺ = 322
¹H-NMR (400 MHz, D₂O) δ (ppm): 2.14 (3H, s), 2.15-2.22 (2H, m), 2.50-2.58 (2H, m), 2.86 (1H, dd, J=9.0 Hz, J=14.0 Hz), 3.03 (1H, dd, J=5.0 Hz, J=14.0 Hz), 3.84 (1H, t, J=6.5 Hz), 3.99 (2H, S), 4.59 (1H, dd, J=5.0 Hz, J=9.0 Hz)

### <Example 5> (reference only)

### <Synthesis of other peptides>

γ-Glu-Met(O), γ-Glu-Val-NH₂, γ-Glu-Val-ol, γ-Glu-Ser, γ-Glu-Tau, γ-Glu-Cys(S-Me)(O), γ-Glu-t-Leu, γ-Glu-Cys(S-allyl)-Gly, and γ-Glu-Cys(S-Me) were synthesized in a manner similar to those of Examples 3 and 4.

### <Example 6>

### <Evaluation of calcium receptor activation activity>

For evaluation of calcium receptor activation activity, a Ca ion concentration-dependent Cl ionic current measuring method using a *Xenopus laevis* oocyte expression system was used. If each activator is added to *Xenopus laevis* oocytes expressing the calcium receptor, intracellular Ca ions increase. Then, the Ca ion concentration-dependent Cl channel opens, and the intracellular current value changes as an ionic current.
By measuring the change in this intracellular current value, whether the calcium receptor activation activity is present or not can be known.

Specifically, the abdomen of *Xenopus laevis* was opened, and an egg batch was taken out and treated with a 1% collagenase solution at 20°C for 2 hours to obtain individual oocytes. Into the oocytes, 50 nl of 1 µg/µl receptor cRNA or 50 nl of sterilized water per one oocyte was introduced by using a micro glass capillary, and the oocytes were cultured at 18°C for 2 or 3 days. For the culture, a solution obtained by adding 2 mM pyruvic acid, 10 U/ml of penicillin, and 10 µg/ml of streptomycin to the solution mentioned in Example 2 was used. After the culture, a test solution was added to the oocytes introduced with cRNA or sterilized water.
Electrophysiological measurement was performed by using an amplifier, Geneclamp500 (Axon), and recording software, AxoScope 9.0 (Axon). The oocytes were voltage-clamped at - 70 mV by the double electrode voltage clamp method, and the intracellular current was measured via the Ca ion concentration-dependent Cl ion channel. The maximum value of the intracellular current was considered as the response current value.

### <Example 7>

### <Evaluation of calcium receptor activation activity of calcium>

The calcium receptor activation activity of calcium was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, calcium was added (2 mM, 5 mM, 10 mM, 20 mM), and Ca ion concentration-dependent Cl response current was measured. The results are shown in Fig. 1. From these results, it was confirmed that cRNA of the calcium receptor introduced into the oocytes were functionally expressed. Furthermore, since the oocytes introduced with water did not respond to even high concentration calcium, it was confirmed that the calcium receptor is not expressed in the oocytes themselves.

### <Example 8>

### <Evaluation of calcium receptor activation activity of L-amino acids>

Calcium receptor activation activity of L-amino acids was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, alanine (10 mM), arginine (10 mM), asparagine (10 mM), aspartic acid (10 mM), cysteine (10 mM), glutamine (10 mM), glutamic acid (10 mM), glycine (10 mM), histidine (10 mM), isoleucine (10 mM), leucine (10mM), lysine (10 mM), methionine (10 mM), phenylalanine (10 mM), proline (10 mM), seine (10 mM), threonine (10 mM), tryptophan (10 mM), tyrosine (10 mM), valine (10 mM), ornithine (10 mM), taurine (10 mM), or hydroxyproline (10 mM) was added, and Ca ion concentration-dependent Cl response current was measured. The results are shown in Fig. 2. By these results, it was demonstrated that cysteine, histidine, phenylalanine, tryptophan, and tyrosine had definite calcium receptor activation activity. As for the aforementioned amino acids, the activation activity was reported in Proc. Natl. Acad. Sci. USA, 2000 Apr. 25, 97(9):4814-9.

### <Example 9>

### <Evaluation of calcium receptor activation activity of D-cysteine>

Calcium receptor activation activity of D-cysteine was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, D-cysteine (10 mM), L-cysteine (10 mM), D-tryptophan (10 mM), or L-tryptophan (10 mM) was added, and Ca ion concentration-dependent Cl response current was measured. The results are shown in Fig. 3. By these results, it was demonstrated that D-cysteine had definite calcium receptor activation activity.

### <Example 10>

### <Evaluation of calcium receptor activation activity of peptides>

Calcium receptor activation activity of peptides was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, γ-Glu-Cys-Gly (50 µM), γ-Glu-Cys(SNO)-Gly (50 µM), γ-Glu-Ala (50 µM), γ-Glu-Gly (500 µM), γ-Glu-Cys (50 µM), γ-Glu-Met (500 µM), γ-Glu-Thr (50 µM), γ-Glu-Val (50 µM), γ-Glu-Orn (500 µM), Asp-Gly (1 mM), Cys-Gly (1 mM), Cys-Met (1 mM), Glu-Cys (50 µM), Gly-Cys (500 µM), or Leu-Asp (1 mM) was added, and Ca ion concentration-dependent Cl response current was measured. The results are shown in Fig. 4. By these results, it was demonstrated that the aforementioned peptides had definite calcium receptor activation activity.

### <Example 11>

### <Evaluation of calcium receptor activation activity of peptides>

Calcium receptor activation activity of peptides was evaluated in the same manner as that of Example 10. Each of the peptides shown in Table 2 was added to voltage-clamped oocytes at 1000 µM, 300 µM, 100 µM, 30 µM, 10 µM, 3 µM, 1 µM, 0.3 µM, and 0.1 µM, and Ca ion concentration-dependent Cl response current was measured. The lowest concentration with which current was detected was shown in Table 2 as the activity. From these results, it became clear that these 32 kinds of peptides had calcium receptor activation activity.

**Table 2**

| No. | Peptide | Activity |
|---|---|---|
| 1 | γ-Glu-Met(O) | 1000µM |
| 2 | γ-Glu-Val-Val | 1000µM |
| 3 | γ-Glu-Val-Glu | 1000µM |
| 4 | γ-Glu-Val-Lys | 1000µM |
| 5 | γ-Glu-Val-Arg | 1000µM |
| 6 | γ-Glu-Val-Asp | 1000µM |
| 7 | γ-Glu-Val-Met | 1000µM |
| 8 | γ-Glu-Val-Thr | 1000µM |
| 9 | γ-Glu-γ-Glu-Val | 1000µM |
| 10 | γ-Glu-Val-NH2 | 1000µM |
| 11 | γ-Glu-Val-ol | 1000µM |
| 12 | γ-Glu-Ser | 300µM |
| 13 | γ-Glu-Tau | 300µM |
| 14 | γ-Glu-Cys(S-Me)(O) | 300µM |
| 15 | γ-Glu-Val-His | 100µM |
| 16 | γ-Glu-Val-Orn | 100µM |
| 17 | γ-Glu-Leu | 100µM |
| 18 | γ-Glu-Ile | 100µM |
| 19 | γ-Glu-t-Leu | 100µM |
| 20 | γ-Glu-Cys(S-allyl)-Gly | 100µM |
| 21 | γ-Glu-Val-Asn | 30µM |
| 22 | γ-Glu-Gly-Gly | 30µM |
| 23 | γ-Glu-Val-Phe | 30µM |
| 24 | γ-Glu-Val-Ser | 30µM |
| 25 | γ-Glu-Val-Pro | 30µM |
| 26 | γ-Glu-Ser-Gly | 30µM |
| 27 | γ-Glu-Cys(S-Me) | 30µM |
| 28 | γ-Glu-Val-Cys | 10µM |
| 29 | γ-Glu-Val-Gln | 10µM |
| 30 | γ-Glu-Abu-Gly | 3µM |
| 31 | γ-Glu-Cys(S-Me)-Gly | 3µM |
| 32 | γ-Glu-Val-Gly | 0.1µM |

### <Example 12> (reference only)

### <Kokumi-imparting activity of peptides and amino acids>

Typical examples are selected from the peptides and amino acids, those calcium receptor activation activity was confirmed: γ-Glu-X-Gly (X represents Cys(SNO), Cys(S-allyl), Gly, Cys(S-Me), Abu, or Ser), γ-Glu-Val-Y (Y represents Gly, Val, Glu, Lys, Phe, Ser, Pro, Arg, Asp, Met, Thr, His, Orn, Asn, Cys, or Gln), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu and γ-Glu-Cys(S-Me), and whether they have kokumi-imparting activity or not was determined by a sensory evaluation test.

The sensory evaluation test was performed as follows.
To distilled water containing sodium glutamate (0.05 g/dl), inosine monophosphate (0.05 g/dl), and calcium chloride (1 mM), alliin (S-allyl-cysteine sulfoxide: cohtrol experiment of Kokumi-imparting activity) , γ-Glu-Cys-Gly, γ-Glu-Cys, γ-Glu-Ala, or γ-Glu-Val was mixed as a sample in an amount of 0.2 g/dl, and whether they had kokumi-imparting activity or not was determined. Sample solutions that became acidic after dissolution of the samples were adjusted to pH 6.8 to 7.2 with NaOH before use. The results.are shown in Table 3.

**Table 3**

| Kokumi-imparting activity of calcium receptor activators | |
|---|---|
| Calcium, receptor activator | Kokumi-imparting activity |
| γGlu-Cys-Gly | + |
| γGlu-Cys | + |
| γGlu-Ala | + |
| γGlu-Val | + |

### <Example 13> (reference only)

### <Kokumi-imparting activity of peptides>

Intensity of kokumi-imparting activity of each peptide, those calcium receptor activation activity was confirmed, was measured by a quantitative sensory evaluation test.

The quantitative sensory evaluation test was performed as follows. To distilled water containing sodium glutamate (0.05 g/dl), inosine monophosphate (0.05 g/dl), and sodium chloride (0.5 g/dl), γ-Glu-Cys-Gly (glutathione), γ-Glu-Ala, γ-Glu-Met, or γ-Glu-Val was mixed as a sample in an amount of 0.1 g/dl, and intensity of kokumi-imparting activity was measured. Sample solutions that became acidic after dissolution of the samples were adjusted to pH 6.8 to 7.2 with NaOH before use. Evaluation was represented with sensory evaluation scores based on the control sample (0 point) and the sample added with glutathione (3 points), and the test was performed with n = 3. The results are shown in Table 4.

**Table 4**

| Sample | Concentration (g/dl) | Intensity of kokumi | | Evaluation remarks |
|---|---|---|---|---|
| | | First and middle taste | Aftertaste | |
| Control | - | 0 | 0 | - |
| γ-Glu-Cys-Gly | 0.1 | 3.0 | 3.0 | Thickness, growth (mouthfulness), and continuity were enhanced. |
| γ-Glu-Ala | 0.1 | 0.5 | 0.2 | Although the effect was weak, thickness was slightly enhanced. |
| γ-Glu-Met 0.1 | | 1.5 | 0.4 | Thickness, and growth (mouthfulness) were slightly enhanced. |
| γ-Glu-Val | 0.1 | 3.0 | 1.0 | Thickness, and growth (mouthfulness) were enhanced mainly for first and middle tastes |

### <Example 14> (reference only)

### <Kokumi-imparting activity of peptides>

Intensity of kokumi-imparting activity of each peptide, those calcium receptor activation activity was confirmed, was measured by a quantitative sensory evaluation test.

The quantitative sensory evaluation test was performed as follows. To distilled water containing sodium glutamate (0.05 g/dl), inosine monophosphate (0.05 g/dl), and sodium chloride (0.5 g/dl), γ-Glu-Cys-Gly (glutathione), γ-Glu-Cys, γ-Glu-Val, or γ-Glu-Val-Gly was mixed as a sample in an amount of 0.1 g/dl, or 0.01 g/dl as required, and intensity of kokumi-imparting activity was measured. Sample solutions that became acidic after dissolution of the samples were adjusted to pH 6.8 to 7.2 with NaOH before use. Evaluation was represented with sensory evaluation scores based on the control sample (0 point) and the sample added with glutathione (3 points), and the test was performed with n = 5. The results are shown in Table 5.

**Table 5**

| Sample | Concentration (g/dl) | Intensity of kokumi | | Evaluation remarks |
|---|---|---|---|---|
| | | First and middle taste | Aftertaste | |
| Control | - | 0 | 0 | - |
| γ-Glu-Cys-Gly | 0.1 | 3.0 | 3.0 | Thickness, growth (mouthfulness), and continuity were enhanced. |
| γ-Glu-Cys | 0.1 | 2.0 | 2.0 | The effect was slightly weaker, but substantially equivalent compared with γGlu-Cys-Gly |
| γ-Olu-Val | 0.1 | 3.0. | 1.0 | Thickness, and growth (mouthfulness) were enhanced mainly for first and middle tastes |
| γ-Glu-Val-Gly | 0.1 | * | * | * |
| γ-Glu-Val-Gly | 0.01 | 3.0 | 3.0 | Thickness, and continuity were mainly enhanced. Total taste was enhanced |

| | | | | |
|---|---|---|---|---|
| *Unmeasurable: Kokumi-imparting activity was too strong and could not be measured by the sensory evaluation. | | | | |

### <Example 15> (reference only)

### <Kokumi-imparting activity of peptides>

Intensity of kokumi-imparting activity of each peptide, those calcium receptor activation activity was confirmed, was measured by a quantitative sensory evaluation test.

The quantitative sensory evaluation test was performed as follows. To distilled water containing sodium glutamate (0.05 g/dl), inosine monophosphate (0.05 g/dl), and sodium chloride (0.5 g/dl), γ-Glu-Cys-Gly (glutathione), γ-Glu-Abu-Gly, or γ-Glu-Val-Gly was mixed as a sample in an amount of 0.1 g/dl or 0.01 g/dl, and intensity of kokumi-imparting activity was measured. Sample solutions that became acidic after dissolution of the samples were adjusted to pH 6.8 to 7.2 with NaOH before use. Evaluation was represented with sensory evaluation scores based on the control sample (0 point) and the sample added with glutathione (3 points), and the test was performed with n = 12. The results are shown in Table 6.

**Table 6**

| Sample | Concentration (g/dl) | Intensity of kokumi | | Evaluation remarks |
|---|---|---|---|---|
| | | First and middle taste | Aftertaste | |
| Control | - | 0 | 0 | - |
| γGlu-Cys-Gly | 0.1 | 3.0 | 3.0 | Thickness, growth (mouthfulness), and continuity were enhanced. |
| γGlu-Abu-Gly | 0.01 | 3.0 | 2.0 | Thickness, and growth (mouthfulness) were enhanced mainly for first and middle tastes. |
| γGlu-Val-Gly | 0.01 | 3.0 | 3.0 | Thickness, and continuity were mainly enhanced. Total taste was enhanced. |

### <Example 16> (reference only)

### <Activity of peptides on basic tastes>

Intensity of activity on basic tastes of each peptide, those calcium receptor activation activity was confirmed, was measured by a quantitative sensory evaluation test.

The quantitative sensory evaluation test was performed as follows. To distilled water containing sodium glutamate (0.2 g/dl) as a umami standard solution, sucrose (5 g/dl) as a sweet taste standard solution, sodium chloride (0.7 g/dl) as a salty taste standard solution, or citric acid (0.05 g/dl) as a sour taste standard solution, γ-Glu-Cys-Gly (glutathione) or γ-Glu-Val-Gly was mixed as a sample at a concentration of 0.0001 to 1 g/dl, and intensity of activity on basic tastes was measured for each sample.

Sample solutions that became acidic after dissolution of the samples with respect to the standard solutions without the samples were adjusted with NaOH to pH not lower or higher by 0.2 than pH of the standard solutions before use. Evaluation was represented with the following sensory evaluation scores: 0 point for control sample, 1 point for fairly intense activity to control, and 2 points for intense activity to control, and the test was performed with n = 12. The samples showed basic taste enhancing activity at concentrations within the aforementioned broad concentration range. The results for typical concentrations are shown in Table 7.

**Table 7**

| Evaluation system | Distilled water | γGlu-Cys-Gly 0.10g/dl | γGlu-Val-Gly 0.005g/dl | γGlu-Val-Gly 0.01g/dl |
|---|---|---|---|---|
| Umami | 0 | 0.7 | 0.7 | 1.5 |
| Sweet taste | 0 | 1.5 | 0.5 | 1.0 |
| Salty taste | 0 | 0.2 | 0.5 | 1.0 |
| Sour taste | 0 | 1.5 | 0.5 | 1.0 |

### <Example 17> (reference only)

### <Activity of peptides for imparting kokumi to consomme soup>

Intensity of activity for imparting kokumi to consomme soup of each peptide, for which calcium receptor activation activity was confirmed, was measured by a quantitative sensory evaluation test.

The quantitative sensory evaluation test was performed as follows. Consomme soup was prepared by dissolving consomme soup powder (35% of sodium chloride, 18% of sodium glutamate, 0.2% of inosine monophosphate, 0.3% of white pepper powder, 0.5% of black pepper power, 8.0% of beef extract powder, 3.0% of white wine powder, 2.0% of celery powder, 8.0% of Chinese cabbage extract powder, 2.5% of onion extract powder, 25.5% of lactose) at a concentration of 5 g/dl. To this consomme soup, γ-Glu-Cys-Gly (glutathione) or γ-Glu-Val-Gly was mixed as a sample at a concentration of 0.0001 to 1 g/dl, and intensity of kokumi-imparting activity was measured for each sample. The consomme soup with the samples that became acidic after dissolution of the samples with respect to the consomme soup without the samples were adjusted with NaOH to pH not lower or higher by 0.2 than pH of the consomme soup without the samples before use. Evaluation was represented with the following sensory evaluation scores: 0 point for control sample, 3 points for intense activity to control, and 5 points for extremely intense activity to control, and the test was performed with n = 12. The samples showed kokumi-imparting activity at concentrations within the aforementioned broad concentration range. The results for typical concentrations are shown in Table 8.

**Table 8**

| Sample | Concentration (g/dl) | Intensity of kokumi | | Evaluation remarks |
|---|---|---|---|---|
| | | First and middle taste | Aftertaste | |
| Control | - | 0 | 0 | - |
| yGlu-Cys-Gly | 0.01 | 3.0 | 3.0 | Thickness, growth (mouthfulness), and continuity were enhanced. |
| γGlu-Val-Gly | 0.0005 | 2.5 | 3.0 | Thickness and growth (mouthfulness) were mainly enhanced from first and middle taste. |
| γGlu-Val-Gly | 0.001 | 3.0 | 3.5 | Thickness and growth (mouthfulness) were mainly enhanced from first and middle taste. |
| γGlu-Val-Gly | 0.01 | 5.0 | 5.0 | Thickness and continuity were mainly enhanced. Total taste was enhanced. |
| γGlu-Val-Gly | 0.1 | 5.0 | 5.0 | Thickness and continuity were mainly enhanced. Total taste was enhanced. |

### <Example 18> (reference only)

### <Activity of peptides for imparting kokumi to Japanese clear soup>

Intensity of activity for imparting kokumi to clear soup of each peptide, for which calcium receptor activation activity was confirmed, was measured by a quantitative sensory evaluation test.

The quantitative sensory evaluation test was performed as follows. Japanese clear soup was prepared by adding 0.5 g/dl of soy sauce and 0.6 g/dl of sodium chloride to bonito kelp stock (solution obtained by adding 5 g of dried kelp to 3 L of water, heating the water, adding 25 g of dried bonito flakes just before boiling, and then filtering the water containing kelp and bonito flakes). To this clear soup, γ-Glu-Cys-Gly (glutathione) or γ-Glu-Val-Gly was mixed as a sample at a concentration of 0.0001 to 1 g/dl, and intensity of kokumi-imparting activity was measured for each sample. The clear soup with the samples that became acidic after dissolution of the samples with respect to the clear soup without the samples were adjusted with NaOH to pH not lower or higher by 0.2 than pH of the clear soup without the samples before use. Evaluation was represented with the following sensory evaluation scores: 0 point for control sample, 3 points for intense activity to control, and 5 points for extremely intense activity to control, and the test was performed with n = 12. The samples showed kokumi-imparting activity at concentrations within the aforementioned broad concentration range. The results for typical concentrations are shown in Table 9.

**Table 9**

| Sample | Concentration (g/dl) | Intensity of kokumi | | Evaluation remarks |
|---|---|---|---|---|
| | | First and middle taste | Aftertaste | |
| Control | - | 0 | 0 | - |
| γGlu-Cys-Gly | 0.01 | 2.0 | 2.0 growth (mouthfulness) | Thickness and were enhanced. |
| γGlu-Val-Gly | 0.0005 | 2.5 | 3.0 | Thickness, growth (mouthfulness) and continuity were mainly enhanced from first and middle taste. |
| γGlu-Val-Gly | 0.001 | 3.5 | 4.0 | Thickness, growth (mouthfulness) and continuity were mainly enhanced from first and middle taste. |
| γGlu-Val-Gly | 0.01 | 5.0 | 5.0 | Thickness, and continuity were mainly enhanced. Total taste was enhanced. |
| γGlu-Val-Gly | 0.1 | 5.0 | 5.0 | Thickness, and continuity were mainly enhanced. Total taste was enhanced. |

### <Example 19> (reference only)

### <Activity of peptides for imparting kokumi to corn soup>

Intensity of activity for imparting kokumi to corn soup of each peptide, for which calcium receptor activation activity was confirmed, was measured by a quantitative sensory evaluation test.

The quantitative sensory evaluation test was performed as follows. To commercially available corn soup, y-Glu-Cys-Gly (glutathione) or y-Glu-Val-Gly was mixed as a sample at a concentration of 0.0001 to 1 g/dl, and intensity of kokumi-imparting activity was measured for each sample. The corn soup witch the samples that became acidic after dissolution of the samples with respect to the corn soup without the samples were adjusted with NaOH to pH not lower or higher by 0.2 than pH of the corn soup without the samples before use. Evaluation was represented with the following sensory evaluation scores: 0 point for control sample, 3 points for intense activity to control, and 5. points for extremely intense activity to control, and the test was performed with n = 12. The samples showed kokumi-imparting activity at concentrations within the aforementioned broad concentration range. The results for typical concentrations are shown in Table 10.

**Table 10**

| Sample | Concentration (g/dl) | Intensity of kokumi | | Evaluation remarks |
|---|---|---|---|---|
| | | First and middle taste | Aftertaste | |
| Control | - | 0 | 0 | - |
| γGlu-Cys-Gly | 0.01 | 3.0 | 3.0 | Richness, thickness and growth (mouthfulness) were enhanced. |
| γGlu-Val-Gly | 0.0005 | 2.5 | 3.0 | Sweet taste, growth (mouthfulness) and continuity were mainly enhanced from first and middle taste. |
| γGlu-Val-Gly | 0.001 | 3.5 | 4.0 | Sweet taste, growth (mouthfulness) and continuity were mainly enhanced from first and middle taste. |
| γGlu-Val-Gly | 0.01 | 4.5 | 5.0 | Growth (mouthfulness) and continuity were mainly enhanced. Total taste was enhanced. |
| γGlu-Val-Gly | 0.1 | 5.0 | 5.0 | Growth (mouthfulness) and continuity were mainly enhanced. Total taste was enhanced. |

### <Example 20> (reference only)

### <Activity of peptides for imparting kokumi to curry sauce>

Intensity of activity for imparting kokumi to curry sauce of each peptide, for which calcium receptor activation activity was confirmed, was measured by a quantitative sensory evaluation test.

The quantitative sensory evaluation test was performed as follows. To curry sauce prepared in a conventional manner by using commercially available curry roux, γ-Glu-Cys-Gly (glutathione) or γ-Glu-Val-Gly was mixed as a sample at a concentration of 0.0001 to 1 g/dl, and intensity of kokumi-imparting activity was measured for each sample. The curry sauces with the samples that became acidic after dissolution of the samples with respect to the curry soup without the samples were adjusted with NaOH to pH not lower or higher by 0.2 than pH of the curry soup without the samples before use. Evaluation was represented with the following sensory evaluation scores: 0 point for control sample, 3 points for intense activity to control, and 5 points for extremely intense activity to control, and the test was performed with n = 12. The samples showed kokumi-imparting activity at concentrations within the aforementioned broad concentration range. The results for typical concentrations are shown in Table 11.

**Table 11**

| Sample | Concentration (g/dl) | Intensity of kokumi | | Evaluation remarks |
|---|---|---|---|---|
| | | First and middle taste | Aftertaste | |
| Control | - | 0 | 0 | - |
| γGlu-Cys-Gly | 0.01 | 3.0 | 3.0 | Richness, thickness and continuity were enhanced. |
| γGlu-Val-Gly | 0.001 | 2.5 | 3.0 | Mildness, richness and growth (mouthfulness) were mainly enhanced. |
| γGlu-Val-Gly | 0.005 | 3.5 | 4.0 | Mildness, richness and growth (mouthfulness) were mainly enhanced. |
| γGlu-Val-Gly | 0.01 | 5.0 | 5.0 | Richness and continuity were mainly enhanced. Total taste was enhanced. |
| γGlu-Val-Gly | 0.1 | 5.0 | 5.0 | Richness and continuity were mainly enhanced. Total taste was enhanced. |

### <Example 21> (reference only)

### <Kokumi-imparting activity observed when peptides and additives such as known calcium receptor activators were used in combination>

Intensity of kokumi-imparting activity of each peptide for which calcium receptor activation activity was confirmed, used with an additive such as known calcium receptor activators in combination was measured by a quantitative sensory evaluation test.

The quantitative sensory evaluation test was performed as follows. To distilled water containing sodium glutamate (0.05 g/dl), inosine monophosphate (0.05 g/dl), and sodium chloride (0.5 g/dl), 0.0001 to 1 g/dl of γ-Glu-Cys-Gly (glutathione) or γ-Glu-Val-Gly, or either of these peptides and another calcium receptor activator (calcium lactate, protamine or polylysine) or GABA (addition concentration: 0.0001 to 1 g/dl) were mixed, and intensity of kokumi-imparting activity was measured. Sample solutions that became acidic after dissolution of the samples were adjusted to pH 6.8 to 7.2 with NaOH before use. Evaluation was represented with the following sensory evaluation scores: 0 point for control sample, 3 points for intense activity (as intensity of 0.05 g/dl γ-Glu-Cys-Gly and 0.005 g/dl γ-Glu-Val-Gly), and 6 points for extremely intense activity (as twice intensity of 0.05 g/dl γ-Glu-Cys-Gly and 0.005 g/dl γ-Glu-Val-Gly), and the test was performer with n = 12. The samples showed kokumi-imparting activity at concentrations within the aforementioned broad concentration range. The results.for typical concentrations are shown in Table 12. As a result, existing compound having kokumi-imparting activity, glutathione, also show improved kokumi-imparting activity as well as kokumi-imparting agent of the present invention when used with existing calcium receptor activator or the like such as calcium.

**Table 12**

| Peptide sample | Concentration (g/dl) | Additive | Concentration (g/dl) | Intensity of kokumi | | Evaluation remarks |
|---|---|---|---|---|---|---|
| | | | | First and middle taste | After taste | |
| - | - | - | - | 0 | 0 | |
| γGlu-Cys-Gly | 0.05 | - | - | 3.0 | 3.0 | Thickness, growth (mouthfulness) and continuity |
| γGlu-Val-Gly | 0.005 | - | - | 3.0 | 3.0 | Thickness and continuity |
| - | - | Calcium lactate | 0.25 | 0.5 | 0.5 | Thickness |
| - | - | Protamine | 0.005 | 1.5 | 1.0 | Growth (mouthfulness) |
| - | - | Polylysine | 0.001 | 0.5 | 0.5 | Thickness |
| - | - | GABA | 0.025 | 0.5 | 0.5 | Thickness |
| γGlu-Cys-Gly | 0.05 | Calcium lactate | 0.25 | 3.5 | 4.0 | Thickness, growth (mouthfulness) and continuity |
| γGlu-Cys-Gly | 0.05 | Protamine | 0.005 | 4.5 | 4.5 | Thickness, growth (mouthfulness) and continuity |
| γGlu-Cys-Gly | 0.05 | Polylysine | 0.001 | 3.5 | 4.0 | Thickness, growth (mouthfulness) and continuity |
| γGlu-Cys-Gly | 0.05 | GABA | 0.025 | 4.5 | 4.5 | Thickness, growth (mouthfulness) and continuity |
| γGlu-Val-Gly | 0.005 | Calcium lactate | 0.25 | 5.0 | 5.0 | Thickness and continuity |
| γGlu-Val-Gly | 0.005 | Protamine | 0.005 | 4.5 | 4.5 | Thickness, growth (mouthfulness) and continuity |
| γGlu-Val-Gly | 0.005 | Polylysine | 0.001 | 4.5 | 4.5 | Thickness and continuity |
| γGlu-Val-Gly | 0.005 | GABA | 0.025 | 4.5 | 4.5 | Richness and thickness |

### Industrial Applicability

By the present invention, a method for screening for a kokumi-imparting substance utilizing calcium receptor activation as an index has been developed. Hence, so-called high throughput screening can be used, and thus development of a still more highly efficient kokumi substance has become possible.

## Claims

1. A method for screening for a kokumi-imparting substance, i.e. a substance capable of enhancing salty taste, umami, sweet taste, sour taste, or bitter taste, which comprises reacting a calcium receptor and a test substance and detecting calcium receptor activity, to indicate a possible kokumi-imparting effect of the test substances.

## Patentansprüche

1. Verfahren zum Screenen auf eine Kokumi verleihende Substanz, d.h. eine Substanz, die salzigen Geschmack, Umami, süßen Geschmack, sauren Geschmack oder bitteren Geschmack verstärken kann, welches das Umsetzen eines Calciumrezeptors und einer Testsubstanz und das Nachweisen von Calciumrezeptoraktivität umfasst, um eine mögliche Kokumi verleihende Wirkung der Testsubstanz anzuzeigen.

## Revendications

1. Procédé de dépistage d'une substance conférant le kokumi, c'est-à-dire une substance capable d'amplifier la saveur salée, la saveur umami, la saveur sucrée, la saveur acide ou la saveur amère, lequel comprend la mise à réagir d'un récepteur du calcium et d'une substance à tester et la détection de l'activité du récepteur du calcium pour indiquer un possible effet conférant le kokumi des substances à tester.


### SEQUENCE LISTING

<110> Ajinomoto Co., Inc.
<120> Kokumi-imparting agent
<130> C565-C6200
<150> JP2005-325300
   <151> 2005-11-09
<150> US 60/738562
   <151> 2005-11-22
<150> JP2006-188458
   <151> 2006-07-07
<150> US 60/807831
   <151> 2006-07-20
<160> 2
<170> PatentIn version 3.1
<210> 1
   <211> 49
   <212> DNA
   <213> Artificial
<220>
   <223> hCASR_N primer
<400> 1
   actaatacga ctcactatag ggaccatggc attttatagc tgctgctgg 49
<210> 2
   <211> 29
   <212> DNA
   <213> Artificial
<220>
   <223> hCASR_C primer
<400> 2
   ttatgaattc actacgtttt ctgtaacag 29